# DEMANDE DE BREVET EUROPEEN

(11) **EP 1 997 475 A2**
(43) Date de publication de la demande: **03.12.2008**
(21) Numéro de dépôt: 08154421.5
(22) Date de dépôt: 11.04.2008
(51) Int. Cl.: A61K 8/49, A61Q 5/06

(54) **Composition comprenant au moins un dérivé substitué de carbocyanine acétylénique, procédé de traitement des fibres kératiniques la mettant en oeuvre et dispositif**

(30) Priorité: 13.04.2007 FR 0754453
(71) Demandeur: L'Oréal, 75008 Paris (FR)
(72) Inventeur: LAGRANGE, Alain, 77700, COUPVRAY (FR)
(74) Mandataire: Wattremez, Catherine

(57) **Abrégé**

L'invention concerne une composition de coloration de fibres kératiniques humaines comprenant, dans un milieu cosmétiquement acceptable, un ou plusieurs colorants directs cationique comprenant au moins une liaison triple carbone-carbone comme par exemple des colorants de formules (I), (II) ou (III) suivantes :

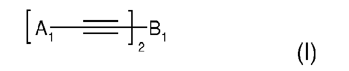

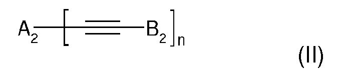

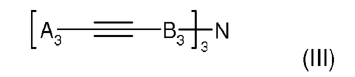

Dans lesquelles A₁, A₂, A₃, indépendamment les uns des autres, correspondent à un groupement hétérocyclique cationique et B₁, B₂, B₃, indépendamment les uns des autres, correspondent à un noyau aromatique particulier ; n pouvant être égal à 1 ou 2.

L'invention concerne de plus un procédé de traitement de fibres kératiniques en particulier humaines, mettant en jeu la composition précitée, ainsi qu'un dispositif à plusieurs compartiments la comprenant.

## Description

L'invention concerne une composition comprenant, dans un milieu cosmétiquement acceptable, au moins un colorant direct cationique de la famille des carbocyanines et portant une liaison acétylénique (liaison triple carbone-carbone).

Elle a de même pour objet un procédé de traitement de fibres kératiniques mettant en jeu une telle composition, ainsi qu'un dispositif la comprenant.

La présente invention a trait au domaine de la coloration des fibres kératiniques et plus particulièrement de la coloration des fibres kératiniques humaines telles que les cheveux.

Il existe de nombreuses compositions et de nombreux procédés pour teindre les fibres kératiniques et, en particulier, les cheveux humains.

Ainsi, il est connu de teindre les fibres kératiniques et, en particulier, les cheveux humains avec des compositions tinctoriales contenant des précurseurs de colorant d'oxydation, en particulier des ortho ou paraphénylènediamines, des ortho ou paraaminophénols, des composés hétérocycliques, tels que des dérivés de diaminopyrazole, appelés généralement « bases d'oxydation ». Les précurseurs de colorants d'oxydation, ou bases d'oxydation, sont des composés incolores ou faiblement colorés qui, associés à des produits oxydants, conduisent par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu, d'une part, au niveau des bases d'oxydation et, d'autre part, au niveau des coupleurs, permet l'obtention d'une riche palette de couleurs.

La coloration, dite « permanente », obtenue grâce à ces colorants d'oxydation, doit, par ailleurs, satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, l'ondulation permanente, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles, tout au long d'une même fibre kératinique, qui peut être en effet différemment sensibilisée, c'est-à-dire abîmée entre sa pointe et sa racine.

Il s'avère, en conclusion, que si les associations base-coupleur classiques permettent d'obtenir une palette étendue de couleurs, elles ne permettent pas de satisfaire aux critères énumérés plus haut et, notamment, conduisent souvent à la génération dans la fibre de produits de couplage variés posant des problèmes de ténacité difficiles à maîtriser, comme par exemple un virage sélectif.

Par ailleurs, la coloration permanente avec des colorants d'oxydation nécessite le plus souvent l'utilisation de systèmes oxydants qui peuvent nuire à l'intégrité des fibres kératiniques (sensibilisation des cheveux).

Une autre manière de teindre les fibres kératiniques, en particulier les cheveux, est d'utiliser des colorants directs.

Les colorants classiques qui sont utilisés sont en particulier des colorants du type nitrés benzéniques, anthraquinoniques, nitropyridiniques, azoïques, azoïques cationiques, xanthéniques, acridiniques aziniques, triarylméthanes, ou des colorants naturels.

Ces colorants qui sont des molécules colorées et colorantes ayant une affinité pour les fibres sont appliqués sur les fibres kératiniques pendant un temps nécessaire à l'obtention de la coloration désirée, puis rincés.

La coloration directe est de ce fait très répandue car elle présente, en outre, certains avantages par rapport aux précurseurs de coloration d'oxydation et, notamment, souvent une meilleure inocuité et l'absence de sensibilisation du cheveu due au processus oxydatif.

Les colorations obtenues sont cependant temporaires ou semi-permanentes, car la nature des interactions qui lient les colorants directs à la fibre kératinique, et leur désorption de la surface et/ou du coeur de la fibre sont responsables de leurs relativement faibles puissance tinctoriale et tenue aux lavages, aux intempéries, ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux.

Les cinétiques d'absorption de ces colorants directs par les fibres kératiniques ne sont par ailleurs pas suffisamment rapides pour obtenir des colorations intenses avec de faibles temps de pause.

Il existe donc un besoin pour une composition de teinture des fibres kératiniques, en particulier des fibres kératiniques humaines et, plus particulièrement, des cheveux, qui soit d'une bonne innocuité vis-à-vis des fibres kératiniques, qui soit peu sélective, qui donne une grande variété de couleurs, puissantes, et qui permette, en outre, d'obtenir une coloration des fibres kératiniques tenace, stable, résistante aux agents extérieurs, tels que la lumière, les intempéries, le lavage, la transpiration, et les frottements et aux traitements ultérieurs, tels que l'ondulation permanente, qui puisse être suffisamment rapide pour le cas échéant l'utilisation de faibles temps de pause.

Il existe encore un besoin pour une composition de teinture qui permette le traitement de toutes sortes de fibres kératiniques, de tous types de cheveux, par exemple des cheveux blancs, même si ces cheveux ont subi préalablement un traitement, tel qu'un traitement de décoloration ou d'ondulation permanente.

Il existe enfin un besoin pour une composition qui réalise la teinture sans qu'il soit nécessaire de réaliser, au préalable, une sensibilisation de la fibre kératinique, du cheveu.

Le but de la présente invention est de fournir une composition de teinture des fibres kératiniques, en particulier des fibres kératiniques humaines et, plus particulièrement, des cheveux, qui réponde, entre autres, aux besoins énumérés ci-dessus.

Le but de la présente invention est encore de fournir une composition de teinture des fibres kératiniques qui ne présente pas les inconvénients, défauts, limitations et désavantages des compositions de teinture de l'art antérieur, qu'il s'agisse des compositions de teinture mettant en oeuvre une association base-coupleur, ou des compositions mettant en oeuvre un colorant direct.

Ce but et d'autres encore sont atteints, conformément à l'invention, par une composition pour la teinture des fibres kératiniques comprenant dans un milieu cosmétique approprié pour la teinture, au moins un colorant direct cationique comportant au moins une liaison triple carbone-carbone ( 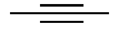 ).

Elle a de même pour objet un procédé de traitement de fibres kératiniques plus particulièrement de fibres kératiniques humaines, dans lequel applique sur lesdites fibres, sèches ou humides, la composition selon l'invention.

Un autre objet de l'invention est constitué par un dispositif à plusieurs compartiments dont au moins l'un comprend la composition selon l'invention et au moins un autre comprend un agent oxydant.

Mais d'autres caractéristiques et avantages de l'invention apparaîtront plus clairement à la lecture de la description et des exemples qui vont suivre.

Dans ce qui va suivre et à moins qu'une mention différente ne soit indiquée, les bornes d'un domaine de valeurs sont comprises comme faisant partie de ce domaine.

Selon la présente invention, on entend par fibres kératiniques humaines, les cheveux, les cils, et les sourcils.

Il est à noter que la composition est appropriée pour le traitement de fibres kératiniques humaines, quelle que soit leur coloration avant traitement et que celle-ci soit naturelle ou obtenue artificiellement.

Comme indiqué auparavant, la composition selon l'invention comprend au moins un colorant direct cationique comprenant au moins une liaison triple carbone-carbone.

Conformément à une variante particulière, la composition de coloration comprend, dans un milieu cosmétiquement acceptable, un ou plusieurs colorants directs de formules (I), (II) ou (III) suivantes : 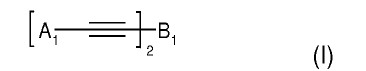 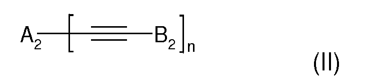 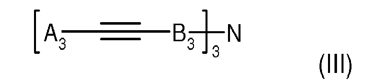 Formules dans lesquelles :
- A₁, A₂, A₃, indépendamment les uns des autres, correspondent à un groupement hétérocyclique cationique de préférence choisi parmi les groupements pyridinium, (benz)imidazolium, (benzo)thiazolium, benzoxazolium, (iso)quinolinium, (benz)indolium, acridinium, thioxanthilium, quinolizinium, phénazinium, pyrrolopyridinium, rattaché à la fonction C≡C ou aux fonctions C≡C au moyen de l'un des atomes de carbone de l'hétérocycle ou du noyau aromatique avec lequel l'hétérocycle est éventuellement condensé ;
- A₁, A₂, A₃, étant éventuellement substitués, en dehors des radicaux portés par les atomes d'azote quaternisés, par un ou plusieurs radicaux, identiques ou non, choisis parmi les atomes d'halogène comme le fluor ou le chlore, les radicaux alkyle en C₁-C₂₅, les radicaux alcoxy (C₁-C₄), les radicaux alkyle(C₁-C₂₀)carbonylamino, les radicaux phényle ;
- le ou les atomes d'azote quaternisés de l'hétérocycle, lorsqu'il sont présents, sont éventuellement porteur(s) d'un radical amino ; d'un radical acyle en C₂-C₂₀ ; d'un radical alkyle en C₁-C₂₅ éventuellement substitué par au moins un radical phényle, phényl-carbonyle, alcoxy(C₁-C₄)carbonyle, sulfo (-SO₃M avec M représentant un atome d'hydrogène, un métal alcalin), éthylényle (-CH=CH₂), acétylényle (-C≡CH) ou phényle-acétylényle (-C≡C-C₆H₅) ;
- B₁, B₂, B₃, indépendamment les uns des autres, correspondent à un noyau aromatique choisi parmi les groupements phényle, anthracényle, naphtényle, rattaché à une ou deux fonctions C≡C ;
- B₁, B₂, B₃, étant éventuellement substitués par un ou plusieurs groupements amino éventuellement porteurs d'un ou deux radicaux identiques ou non choisis parmi les radicaux alkyles en C₁-C₁₈, alkyl(C₁-C₂₀)carbonyle, alkyl(C₁-C₂₀)sulfonyle, phényle éventuellement substitué par un groupement alcoxy en C₁-C₄ ; hydroxyle ; alcoxy en C₁-C₄ ; alkyle en C₁-C₁₀ éventuellement porteur d'un groupement amino ; phénoxy ; sulfo (-SO₃M avec M représentant un atome d'hydrogène, un métal alcalin) ; cyano ; trifluorométhyle ; halogène choisi parmi le chlore, le fluor et le brome ; acétylthio (CH₃-CO-S-) ;
- n est égal à 1 ou 2 ;
- l'électroneutralité des composés étant assurée, si nécessaire, au moyen d'un ou plusieurs anions, identiques ou différents, cosmétiquement acceptables.

Selon un premier mode de réalisation particulier de l'invention, le colorant direct est de formule (I), et le groupement A₁ est un groupement phénazinium.

Selon ce mode de réalisation, le groupement B₁ est de préférence un groupement anthracényle.

Selon un deuxième mode de réalisation de l'invention, le colorant direct est de formule (II).

Dans une première variante, n vaut 1 et le groupement A₂ est de préférence choisi parmi les groupements pyridinium, benzimidazolium, (benzo)thiazolium, benzoxazolium, (iso)quinolinium, (benz)indolium, acridinium, thioxanthilium, quinolizinium, pyrrolopyridinium, éventuellement substitués.

Dans une deuxième variante, n vaut 2 et le groupement A₂ est de préférence un groupement pyridinium.

Quel que soit la variante retenue, les groupements B₂, indépendamment les uns des autres, correspondent à un groupement phényle, éventuellement substitué, naphtalényle. De préférence, les groupements B₂ sont identiques.

Selon un troisième mode de réalisation de l'invention, le colorant direct est de formule (III) et les groupements A₃ sont des groupements pyridinium.

Conformément à ce mode de réalisation, les groupements B₃ sont de préférence des groupements phényle. De préférence, les groupements B₃ sont identiques.

Dans les formules (I), (II) ou (III), le ou les anions cosmétiquement acceptables sont plus particulièrement choisis parmi les halogénures, les sulfates, les bisulfates, les nitrates, les phosphates, les hydrogénophosphates, les dihydrogénophosphates, les carbonates, les bicarbonates, les perchlorates, les sels d'acides mono- ou polycarboxyliques, sulfoniques, saturés ou non, aromatiques ou non, éventuellement substitués par au moins un radical hydroxyle, amino, ou au moins un atome d'halogène ; les phénols.

Conformément à un mode de réalisation particulièrement avantageux de l'invention, le composé direct correspond à l'une des formules suivantes :

| | |
|---|---|
| 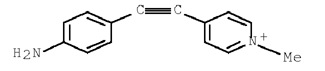 | 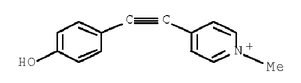 |
| Sel de pyridinium, 4-[(4-aminophenyl)ethynyl]-1-methyl | Sel de pyridinium, 4-[(4-hydroxyphenyl)ethynyl]-1-methyl |
| 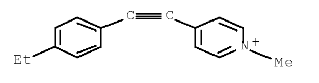 | 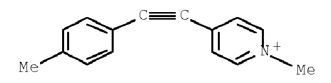 |
| Sel de pyridinium, 4-[(4-ethylphenyl)ethynyl]-1-methyl- | Sel de pyridinium, 1-methyl-4-[(4-methylphenyl)ethynyl] |
| 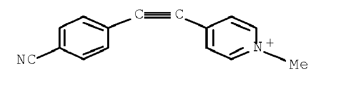 | 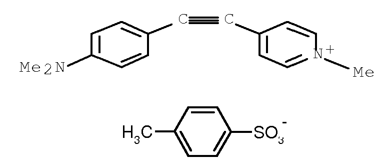 |
| Sel de pyridinium, 4-[(4-cyanophenyl)ethynyl]-1-methyl | Sel d'acide 4-méthylbenzènesulfonique (1:1) et de pyridinium, 4-[[4-(dimethylamino)phenyl]ethynyl]-1-methyl-, monohydraté |
| | 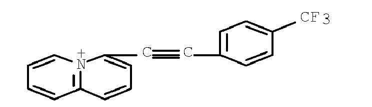 |
| | Sel de quinolizinium, 4-[[4-(trifluoromethyl)phenyl]ethynyl]- |
| 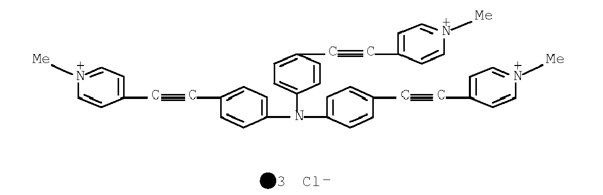 | |
| Trichlorure de pyridinium, 4,4',4"-[nitrilotris(4,1-phenylene-2,1-ethynediyl)]tris[1-methyl-, trichloride | |
| 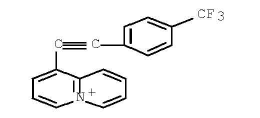 | 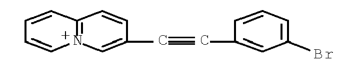 |
| Sel de quinolizinium, 1-[[4-(trifluoromethyl)phenyl]ethynyl]- | Sel de quinolizinium, 3-[(3-bromophenyl)ethynyl]- |
| 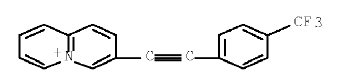 | 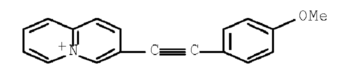 |
| Sel de quinolizinium, 3-[[4-(trifluoromethyl)phenyl]ethynyl]- | Sel de quinolizinium, 3-[(4-methoxyphenyl)ethynyl]- |
| 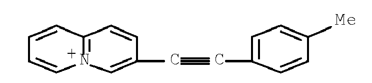 | 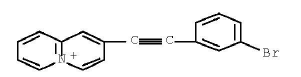 |
| Sel de quinolizinium, 3-[(4-methylphenyl)ethynyl]- | Sel de quinolizinium, 2-[(3-bromophenyl)ethynyl]- |
| 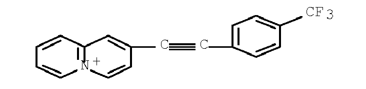 | 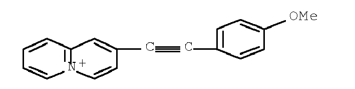 |
| Sel de quinolizinium, 2-[[4-(trifluoromethyl)phenyl]ethynyl] | Sel de quinolizinium, 2-[(4-methoxyphenyl)ethynyl] |
| 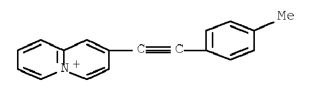 | 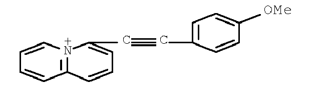 |
| Sel de quinolizinium, 2-[(4-methylphenyl)ethynyl]- | Sel de quinolizinium, 4-[(4-methoxyphenyl)ethynyl]- |
| 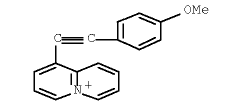 | 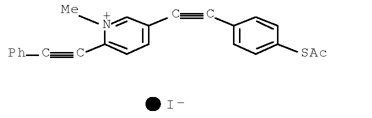 |
| Sel de quinolizinium, 1-[(4-methoxyphenyl)ethynyl]- | Iodure de pyridinium, 5-[[4-(acetylthio)phenyl]ethynyl]-1-methyl-2-(phenylethynyl) |
| 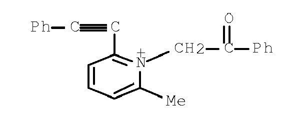 | 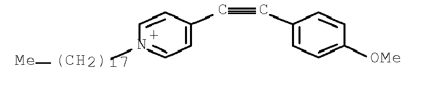 |
| Sel de pyridinium, 2-methyl-1-(2-oxo-2-phenylethyl)-6-(phenylethynyl)- | Sel de pyridinium, 4-[(4-methoxyphenyl)ethynyl]-1-octadecyl- |
| 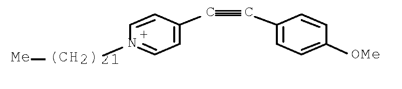 | 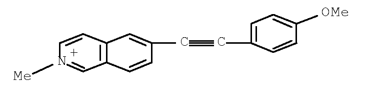 |
| Sel de pyridinium, 1-docosyl-4-[(4-methoxyphenyl)ethynyl]- | Sel d'isoquinolinium, 6-[(4-methoxyphenyl)ethynyl]-2-methyl |
| 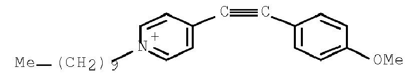 | 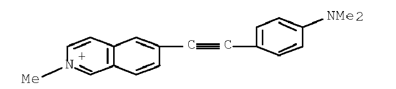 |
| Sel de pyridinium, 1-decyl-4-[(4-methoxyphenyl)ethynyl]- | Sel d'isoquinolinium, 6-[[4-(dimethylamino)phenyl]ethynyl]-2-methyl |
| 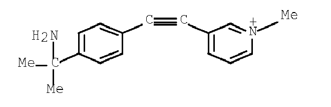 | 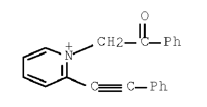 |
| Sel de pyridinium, 3-[[4-(1-amino-1-methylethyl)phenyl]ethynyl]-1-methyl | Sel de pyridinium, 1-(2-oxo-2-phenylethyl)-2-(phenylethynyl)- |
| 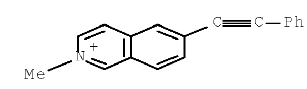 | 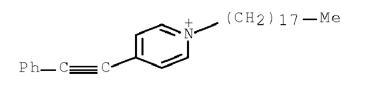 |
| Sel d'isoquinolinium, 2-methyl-6-(phenylethynyl)- | Sel de pyridinium, 1-octadecyl-4-(phenylethynyl)- |
| 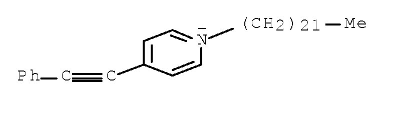 | 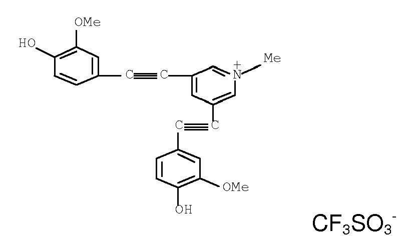 |
| Sel de pyridinium, 1-docosyl-4-(phenylethynyl)- | Sel d'acide trifluorométhanesulfonique (1:1) et de pyridinium, 3,5-bis[(4-hydroxy-3-methoxyphenyl)ethynyl]-1-methyl |
| 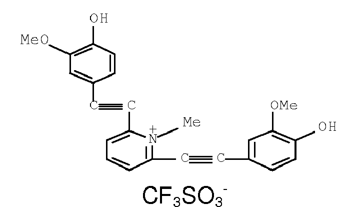 | 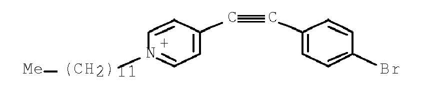 |
| Sel d'acide trifluorométhanesulfonique (1:1) et de pyridinium, 2,6-bis[(4-hydroxy-3-methoxyphenyl)ethynyl]-1-methyl | Sel de pyridinium, 4-[(4-bromophenyl)ethynyl]-1-dodecyl |
| 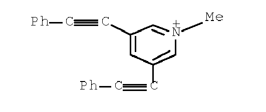 | 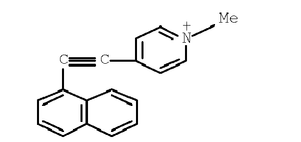 |
| Sel de pyridinium, 1-methyl-3,5-bis(phenylethynyl)- | Sel de pyridinium, 1-methyl-4-(1-naphthalenylethynyl) |
| 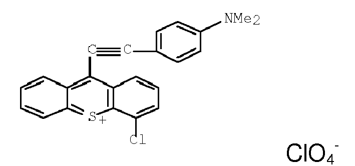 | 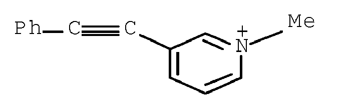 |
| Perchlorate de thioxanthylium, 4-chloro-9-[[4-(dimethylamino)phenyl]ethynyl] | Sel de pyridinium, 1-methyl-3-(phenylethynyl)- |
| 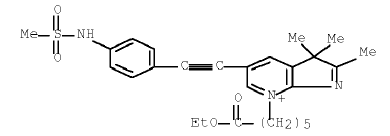 | 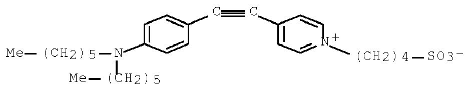 |
| Sel de 3H-Pyrrolo[2,3-b]pyridinium, 7-(6-ethoxy-6-oxohexyl)-2,3,3-trimethyl-5-[[4-[(methylsulfonyl)amino]phenyl]ethynyl | Sel interne de pyridinium, 4-[[4-(dihexylamino)phenyl]ethynyl]-1-(4-sulfobutyl) |
| 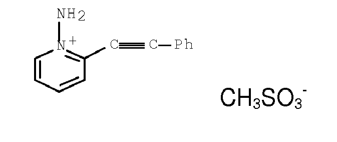 | 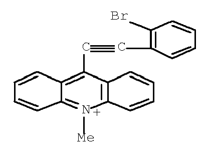 |
| Méthanesulfonate de pyridinium, 1-amino-2-(phenylethynyl) | Sel d'acridinium, 9-[(2-bromophenyl)ethynyl]-10-methyl |
| 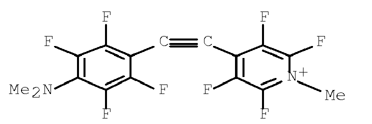 | 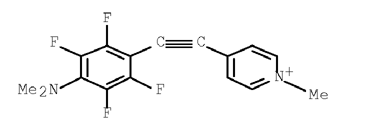 |
| Sel de pyridinium, 4-[[4-(dimethylamino)-2,3,5,6-tetrafluorophenyl]ethynyl]- 2,3,5,6-tetrafluoro-1-methyl | Sel de pyridinium, 4-[[4-(dimethylamino)-2,3,5,6-tetrafluorophenyl]ethynyl]-1-methyl- |
| 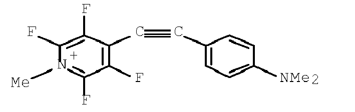 | 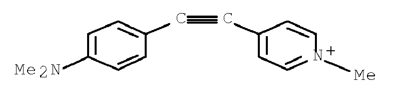 |
| Sel de pyridinium, 4-[[4-(dimethylamino)phenyl]ethynyl]-2,3,5,6-tetrafluoro-1-methyl- | Sel de pyridinium, 4-[[4-(dimethylamino)phenyl]ethynyl]-1-methyl- |
| 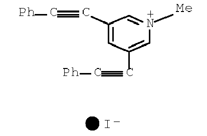 | 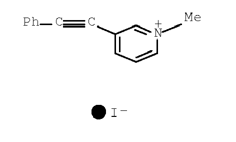 |
| Iodure de pyridinium, 1-methyl-3,5-bis(phenylethynyl) | Iodure de pyridinium, 1-methyl-3-(phenylethynyl) |
| 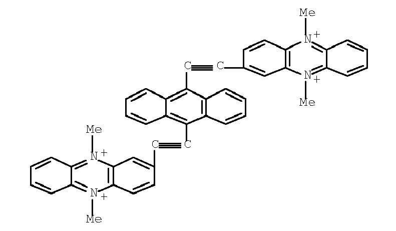 | 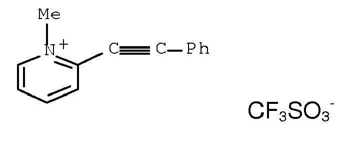 |
| Sel de phenazinium, 2,2'-(9,10-anthracenediyldi-2,1-ethynediyl)bis[5,10-dimethyl | Sel d'acide trifluorométhanesulfonique et de pyridinium, 1-methyl-2-(phenylethynyl) |
| 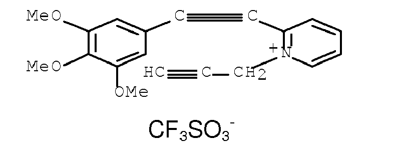 | 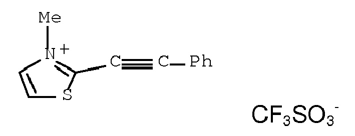 |
| Sel d'acide trifluoromethanesulfonique (1 :1) et de pyridinium, 1-(2-propynyl)-2-[(3,4,5-trimethoxyphenyl)ethynyl] | Sel d'acide trifluoromethanesulfonique (1:1) et de thiazolium, 3-methyl-2-(phenylethynyl) |
| 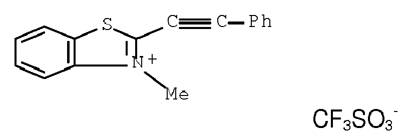 | 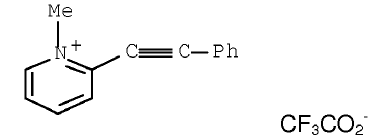 |
| Sel d'acide trifluorométhanesulfonique (1:1) et de benzothiazolium, 3-methyl-2-(phenylethynyl) | Sel d'acide trifluoroacétique(1:1) et de pyridinium, 1-methyl-2-(phenylethynyl) |
| 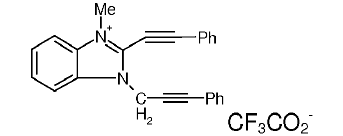 | 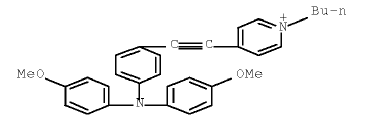 |
| sel d'acide trifluoroacétique (1:1) et de 1H-Benzimidazolium, 1-methyl-2-(phenylethynyl)-3-(3-phenyl-2-propynyl) | Sel de pyridinium, 4-[[4-[bis(4-methoxyphenyl)amino]phenyl]ethynyl]-1-butyl- |
| 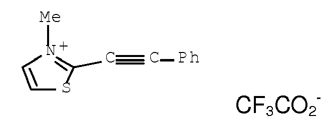 | 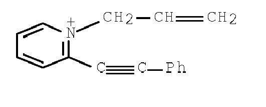 |
| Sel d'acide trifluoroacétique (:1) et de thiazolium, 3-methyl-2-(phenylethynyl) | Sel de pyridinium, 2-(phenylethynyl)-1-(2-propenyl)- |
| 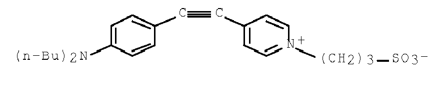 | 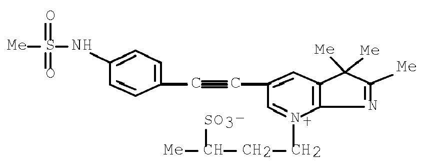 |
| Sel interne de pyridinium, 4-[[4-(dibutylamino)phenyl]ethynyl]-1-(3-sulfopropyl) | Sel interne de 3H-Pyrrolo[2,3-b]pyridinium, 2,3,3-trimethyl-5-[[4-[(methylsulfonyl)amino]phenyl]ethynyl]-7-(3-sulfobutyl) |
| 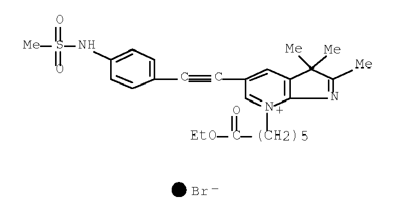 | 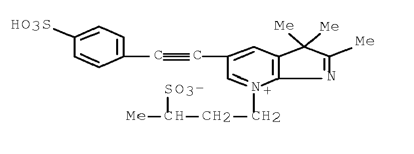 |
| Bromure de 3H-Pyrrolo[2,3-b]pyridinium, 7-(6-ethoxy-6-oxohexyl)-2,3,3-trimethyl-5-[[4-[(methylsulfonyl)amino]phenyl]ethynyl]-, | Sel interne de 3H-Pyrrolo[2,3-b]pyridinium, 2,3,3-trimethyl-7-(3-sulfobutyl)-5-[(4-sulfophenyl)ethynyl] |
| 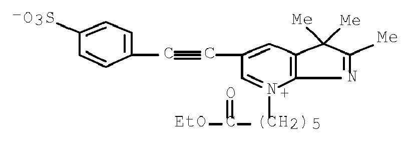 | 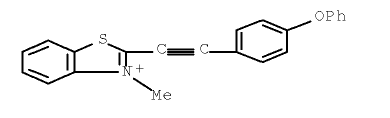 |
| Sel interne de 3H-Pyrrolo[2,3-b]pyridinium, 7-(6-ethoxy-6-oxohexyl)-2,3,3-trimethyl-5-[(4-sulfophenyl)ethynyl] | Sel de benzothiazolium, 3-methyl-2-[(4-phenoxyphenyl)ethynyl]- |
| 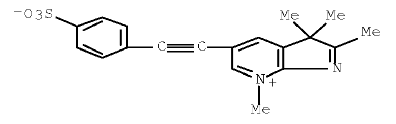 | 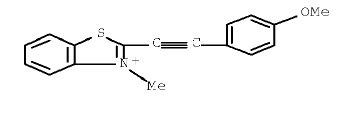 |
| Sel interne de 3H-Pyrrolo[2,3-b]pyridinium, 2,3,3,7-tetramethyl-5-[(4-sulfophenyl)ethynyl] | Sel de benzothiazolium, 2-[(4-methoxyphenyl)ethynyl]-3-methyl |
| 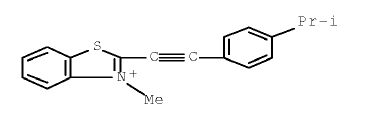 | 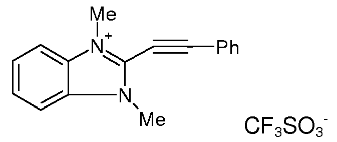 |
| Sel de benzothiazolium, 3-methyl-2-[[4-(1-methylethyl)phenyl]ethynyl]- | Sel d'acide trifluorométhanesulfonique acid (1:1) et de 1H-Benzimidazolium, 1,3-dimethyl-2-(phenylethynyl) |
| 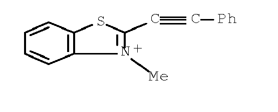 | 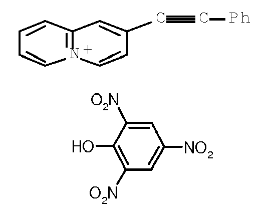 |
| Sel de benzothiazolium, 3-methyl-2-(phenylethynyl)- | Sel de 2,4,6-trinitrophenol (1:1) et de quinolizinium, 2-(phenylethynyl) |
| 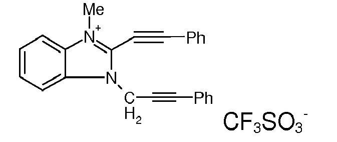 | 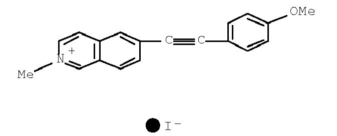 |
| Sel d'acide trifluoromethanesulfonique (1:1) 1H-Benzimidazolium, 1-methyl-2-(phenylethynyl)-3-(3-phenyl-2-propynyl) | Iodure d'Isoquinolinium, 6-[(4-methoxyphenyl)ethynyl]-2-methyl |
| 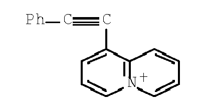 | 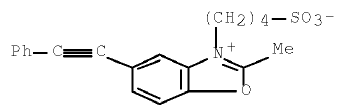 |
| Sel de 2,4,6-trinitrophenol (1:1) et de quinolizinium, 1-(phenylethynyl) | Sel interne de benzoxazolium, 2-methyl-5-(phenylethynyl)-3-(4-sulfobutyl) |
| 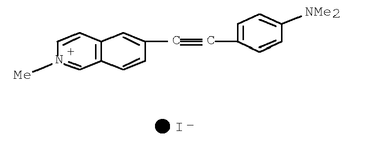 | 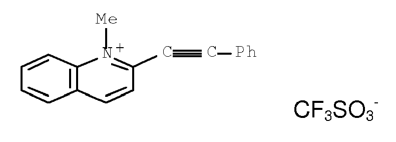 |
| Iodure d'isoquinolinium, 6-[[4-(dimethylamino)phenyl]ethynyl]-2-methyl | Sel d'acide trifluorométhanesulfonique (1:1) et de quinolinium, 1-methyl-2-(phenylethynyl) |
| 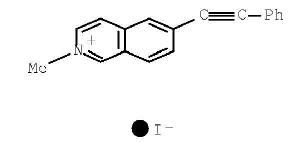 | 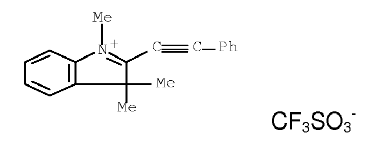 |
| Iodure d'isoquinolinium, 2-methyl-6-(phenylethynyl) | Sel d'acide trifluoromethanesulfonique (1:1) et de 3H-Indolium, 1,3,3-trimethyl-2-(phenylethynyl) |
| 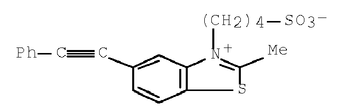 | 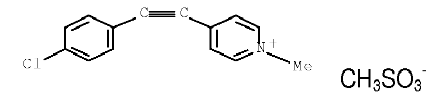 |
| Sel interne de benzothiazolium, 2-methyl-5-(phenylethynyl)-3-(4-sulfobutyl) | Méthylsulfate de pyridinium, 4-[(4-chlorophenyl)ethynyl]-1-methyl-, methyl sulfate |
| 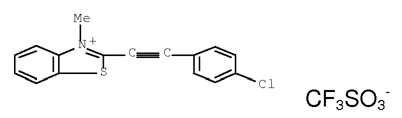 | 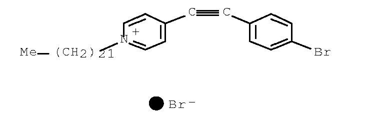 |
| Sel d'acide trifluorométhanesulfonique (1:1) et de b Benzothiazolium, 2-[(4-chlorophenyl)ethynyl]-3-methyl-, | Bromure de pyridinium, 4-[(4-bromophenyl)ethynyl]-1-docosyl |
| 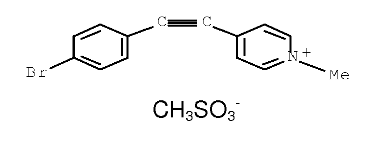 | 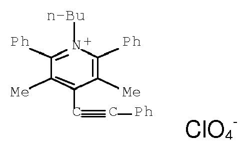 |
| Méthylsulfate de pyridinium, 4-[(4-bromophenyl)ethynyl]-1-methyl- | Perchlorate de pyridinium, 1-butyl-3,5-dimethyl-2,6-diphenyl-4-(phenylethynyl) |
| 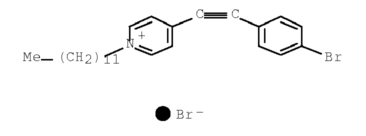 | 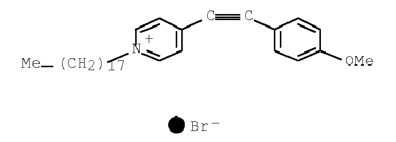 |
| Bromure de pyridinium, 4-[(4-bromophenyl)ethynyl]-1-dodecyl | Bromure de pyridinium, 4-[(4-methoxyphenyl)ethynyl]-1-octadecyl |
| 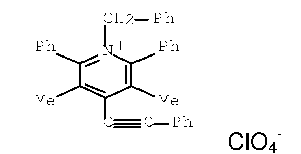 | 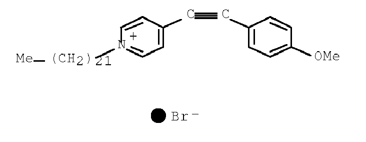 |
| Perchlorate de pyridinium, 3,5-dimethyl-2,6-diphenyl-4-(phenylethynyl)-1-(phenylmethyl) | Bromure de pyridinium, 1-docosyl-4-[(4-methoxyphenyl)ethynyl] |
| 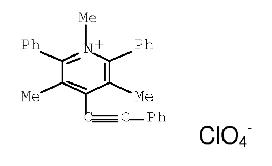 | 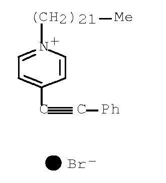 |
| Perchlorate de pyridinium, 1,3,5-trimethyl-2,6-diphenyl-4-(phenylethynyl) | Bromure de pyridinium, 1-docosyl-4-(phenylethynyl) |
| 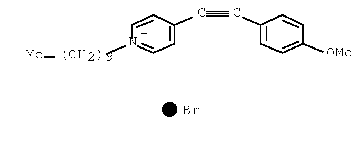 | 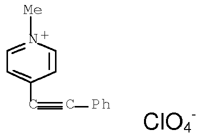 |
| Bromure de pyridinium, 1-decyl-4-[(4-methoxyphenyl)ethynyl] | Perchlorate de 1-Methyl-4-phenylethynylpyridinium |
| 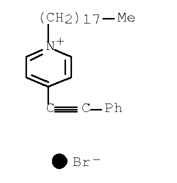 | 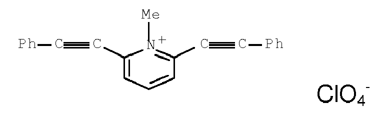 |
| Bromure de pyridinium, 1-octadecyl-4-(phenylethynyl) | Perchlorate de 1-Methyl-2,6-bis(phenylethynyl)pyridinium |
| 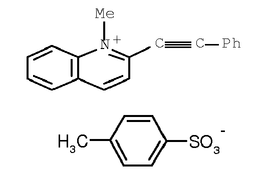 | 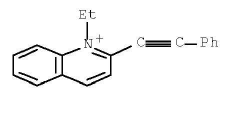 |
| Paratoluènesulfonate de 1-Methyl-2-phenylethynylquinolinium | Perchlorate de 1-Ethyl-2-phenylethynylquinolinium |
| 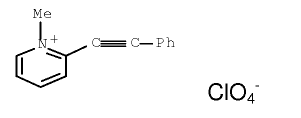 | 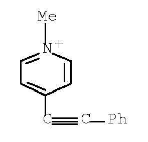 |
| Perchlorate de 1-Methyl-2-phenylethynylpyridinium | Sel de 1-Methyl-4-phenylethynylpyridinium methyl |
| 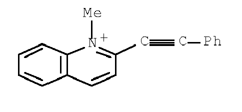 | 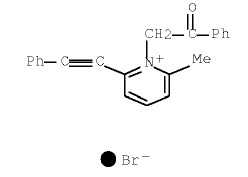 |
| Perchlorate de 1-Methyl-2-phenylethynylquinolinium | Bromure de pyridinium, 2-methyl-1-(2-oxo-2-phenylethyl)-6-(phenylethynyl) |
| 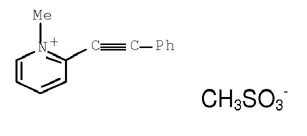 | 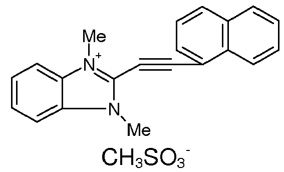 |
| Méthylsulfate de -Methyl-2-phenylethynylpyridinium | Méthylsulfate de 1H-Benzimidazolium, 1,3-dimethyl-2-(1-naphthalenylethynyl) |
| 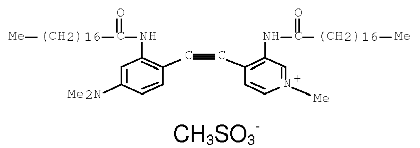 | 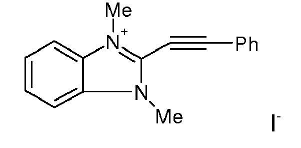 |
| Methyl sulfate de pyridinium, 4-[[4-(dimethylamino)-2-[(1-oxooctadecyl)amino]phenyl]ethynyl]-1-methyl-3-[(1-oxooctadecyl)amino] | Iodure de benzimidazolium, 1,3-dimethyl-2-(phenylethynyl)-, |
| 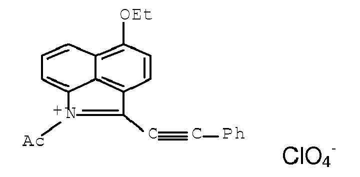 | 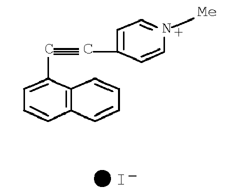 |
| Perchlorate de benz[cd]indolium, 1-acetyl-5-ethoxy-2-(phenylethynyl) | Iodure de pyridinium, 1-methyl-4-(1-naphthalenylethynyl)-, |
| 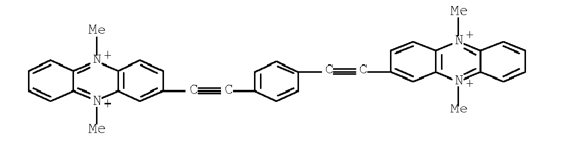 | |
| Sel de phenazinium, 2,2'-(1,4-phenylenedi-2,1-ethynediyl)bis[5,10-dimethyl- | |

Les anions mentionnés dans ce tableau ci-dessus ne sont donnés qu'à titre d'exemple.

Le ou les colorants directs de formules (I), (II) ou (III) représentent plus particulièrement de 0,01 à 20 % en poids, de préférence de 0,1 à 5 % en poids par rapport au poids total de la composition.

La composition peut également comprendre au moins une base d'oxydation.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, Ia 2,6-diéthyl paraphénylènediamine, la 2,3-diméthyl paraphénylènediamine, la N,N-bis-(β-hydroxyéthyl) paraphénylènediamine, Ia 2-chloro paraphénylènediamine, Ia 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, Ia N,N'-bis-(β-hydroxyéthyl) N,N'-bis-(4-aminophényl) tétraméthylènediamine, Ia N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

La ou les bases d'oxydation présentes dans la composition de l'invention sont en général présentes en quantité comprise allant de 0,001 à 20 % en poids environ du poids total de la composition tinctoriale, de préférence allant de 0,005 à 6 %.

Si la composition contient au moins une base d'oxydation, cette dernière est éventuellement et de préférence associée à un ou plusieurs coupleurs. Parmi les coupleurs, on peut utiliser ceux conventionnellement mis en oeuvre pour la teinture de fibres kératiniques. On peut notamment citer les méta-phénylènediamines, les métaaminphénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

Dans la composition de la présente invention, le ou les coupleurs sont généralement présents en quantité comprise allant de 0,001 à 20 % en poids environ du poids total de la composition tinctoriale, de préférence allant de 0,005 à 6%.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La composition tinctoriale conforme à l'invention peut en outre contenir un ou plusieurs colorants directs additionnels autres que les colorants directs de formule (I), (II) ou (III) selon l'invention, pouvant notamment être choisis parmi les colorants nitrés de la série benzénique neutres, acides ou cationiques, les colorants directs azoïques, azométhiniques ou méthiniques neutres, acides ou cationiques, les colorants directs quinoniques et en particulier anthraquinoniques, naphtoquinoniques ou benzoquinoniques neutres, acides ou cationiques, les colorants directs aziniques, les colorants directs triarylméthaniques, les colorants directs indoaminiques les colorants tétraazapentaméthiniques, phénotiaziniques indigoïdes, xanthéniques, phénanthridiniques, phtalocyanines et les colorants directs naturels, seuls ou en mélanges.

Parmi les colorants directs benzéniques utilisables selon l'invention, on peut citer de manière non limitative les composés suivants :
- 1,4-diamino-2-nitrobenzène,
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyioxy-2-β-hydroxyéthyiamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, azométhines ou méthines utilisables selon l'invention on peut citer les colorants cationiques décrits dans les demandes de brevets WO 95/15144, WO 95/01772 et EP 714954 dont le contenu fait partie intégrante de l'invention.

Parmi ces composés, on peut tout particulièrement citer les colorants suivants, ainsi que d'autres sels cosmétiquement acceptables de ces composés :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino- 1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous, An étant défini comme précédemment :

| | |
|---|---|
| 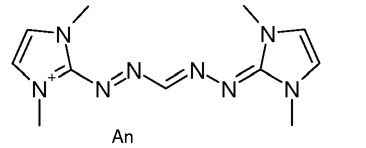 | 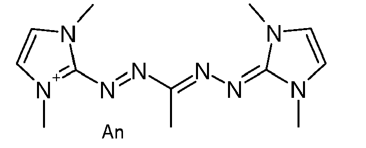 |
| 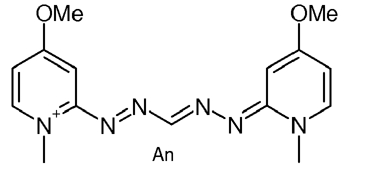 | 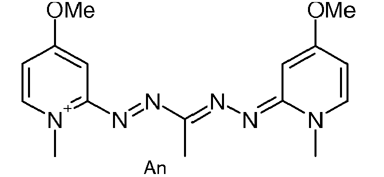 |
| 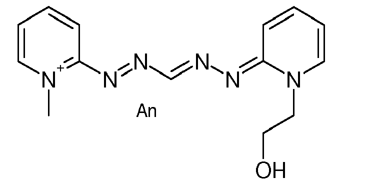 | 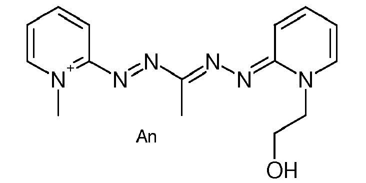 |
| 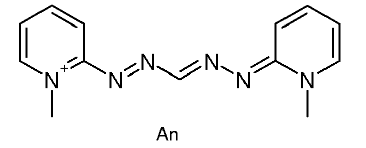 | 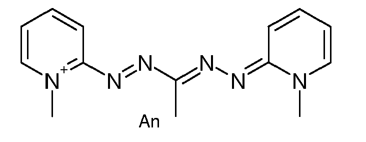 |
| 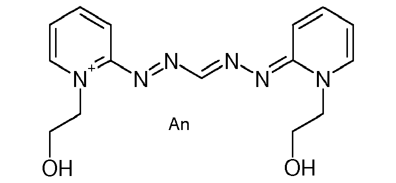 | 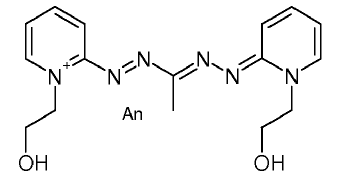 |

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs additionnels différents de ceux de formule (1) représentent de préférence de 0,001 à 20 % en poids environ du poids total de la composition, et encore plus préférentiellement de 0,005 à 10 % en poids environ.

La composition selon l'invention peut également comprendre au moins un agent oxydant classiquement utilisé pour la teinture d'oxydation des fibres kératiniques, comme par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

Le milieu cosmétiquement acceptable est généralement constitué par de l'eau ou par un mélange d'eau et d'au moins un solvant organique.

A titre de solvant organique, on peut par exemple citer, les alcanols, linéaires ou ramifiés, en C₁-C₄, tels que l'éthanol et l'isopropanol ; le glycérol ; les glycols et éthers de glycols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Le ou les solvants peuvent être présents dans des proportions allant de préférence de 1 à 40 % en poids environ par rapport au poids total de la composition tinctoriale, et encore plus préférentiellement de 5 à 30 % en poids environ.

Le pH de la composition conforme à l'invention est généralement compris entre 3 et 12 environ, et de préférence entre 4 et 10 environ. Il peut être ajusté à la valeur désirée au moyen d'agents acidifiants ou alcalinisants habituellement utilisés dans le domaine.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 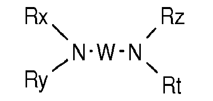 dans laquelle W est un reste propylène éventuellement substitué par un groupement hydroxyle ou un radical alkyle en C₁-C₆ ; Rx, Ry, Rz et Rt, identiques ou différents, représentent un atome d'hydrogène, un radical alkyle en C₁-C₆ ou hydroxyalkyle en C₁-C₆.

Selon un mode de réalisation particulièrement avantageux, la composition comprend un ou plusieurs agents tensioactifs. Ces derniers peuvent être indifféremment choisis, seuls ou en mélanges, parmi des tensioactifs anioniques, amphotères, non ioniques, zwittérioniques et cationiques.

En ce qui concerne les tensioactifs anioniques, on utilise habituellement les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants, seuls ou en mélange :
- les alkylsulfates, les alkyléthersulfates, les alkylamidoéthersulfates, les alkylarylpolyéthersulfates ;
- les alkylsulfonates, les alkylamidesulfonates, les alkyl-arylsulfonates ;
- les alkylsulfosuccinates, les alkyléthersulfosuccinates, les alkylamide-sulfosuccinates ;
- les alkylsulfoacétates ;
- les acylsarconisates ; et les acylglutamates :

- les esters d'alkyle et d'acides polyglycoside-carboxyliques tels que les glucoside-citrates d'alkyle, les polyglycoside-tartrates d'alkyle, et les polyglycoside-sulfosuccinates d'alkyle ;
- les alkylsulfosuccinamates ;
- les acyliséthionates, les N-acyltaurates ; les acyllactylates ;
- les acides d'alkyl-D-galactoside uroniques ;
- les acides alkyléther-carboxyliques polyoxyalkylénés, les acides alkylaryléther-carboxyliques polyoxyalkylénés, les acides alkylamidoéther carboxyliques polyoxyalkylénés ;
le groupe alkyle ou acyle (RCO-) de ces composés comportant de 10 à 24 atomes de carbone et le groupe aryle désignant de préférence un groupe phényle ou benzyle ; le nombre de groupements oxyalkylénés, et de préférence oxyéthylénés, est compris entre 2 et 50.

Pour ce qui concerne les tensioactifs non ioniques, ces derniers peuvent être avantageusement choisis parmi les composés suivants, seuls ou en mélange :
- les alcools gras polyéthoxylés, polypropoxylés ou polyglycérolés,
- les alpha-diols polyéthoxylés, polypropoxylés ou polyglycérolés,
   le nombre de groupements oxyde d'éthylène ou oxyde de propylène allant de 2 à 50 ; le nombre de groupements glycérol allant de 2 à 30 ;
- les condensats d'oxyde d'éthylène et d'oxyde de propylène sur des alcools gras ;
- les amides gras polyéthoxylés ayant de 2 à 30 moles d'oxyde d'éthylène ;
- les amides gras polyglycérolés comportant de 1 à 5 groupements glycérol ;
- les amines grasses polyéthoxylées ayant de 2 à 30 moles d'oxyde d'éthylène ;
- les esters éthoxylés d'acides gras du sorbitane ayant de 2 à 30 moles d'oxyde d'éthylène, les esters d'acides gras du saccharose ;
- les alkylpolyglucosides, les dérivés de N-alkylglucamine ;
   ces composés comprenant au moins une chaîne alkyle ou alcényle comprenant 10 à 24 atomes de carbone.
- les copolymères d'oxyde d'éthylène et d'oxyde de propylène.

Les tensioactifs cationiques entrant dans la composition selon l'invention, peuvent notamment être choisis parmi les composés suivants, seuls ou en mélange :
- les amines grasses primaires, secondaires ou tertiaires éventuellement polyéthoxylées (2 à 30 moles d'oxyde d'éthylène) et leurs sels
- les sels d'ammonium quaternaire tels que les chlorures ou les bromures de tétraalkylammonium, d'alkylamidoalkyltrialkylammonium, de trialkylbenzyl-ammonium, de trialkylhydroxyalkylammonium, d'alkylpyridinium,
- les dérivés d'alkyl-imidazoline ;
   ces composés comprenant au moins une chaîne alkyle comprenant 10 à 24 atomes de carbone.

Enfin, les tensioactifs amphotères peuvent être choisis parmi les composés suivants, seuls ou en mélange :
- les dérivés d'amines aliphatiques secondaires ou tertiaires, dans lesquels le groupe aliphatique est une chaîne linéaire ou ramifiée comportant 10 à 24 atomes de carbone et comprenant au moins un groupe anionique hydrosolubilisant tel que, par exemple, un groupe carboxylate, sulfonate, sulfate, phosphate ou phosphonate,
- les alkylbétaïnes, les sulfobétaïnes, les alkylamidoalkyl(C₆-C₈)bétaïnes, les alkylamidoalkyl(C₆-C₈)sulfobétaïnes ;
ces composés comprenant une au moins chaîne alkyle comprenant de 10 à 24 atomes de carbone.

De préférence, les tensioactifs sont non ioniques, anioniques ou amphotères, et de manière encore plus préférée, non ioniques.

Habituellement, les agents tensioactifs sont présents dans une quantité comprise entre 0,01 et 50 % en poids, de préférence entre 0,1 et 25 % en poids par rapport au poids total de la composition.

La composition cosmétique conforme à l'invention peut également comprendre un ou plusieurs adjuvants utilisés classiquement dans les compositions cosmétiques notamment de teinture des fibres kératiniques humaines, tels que des polymères anioniques, cationiques, non ioniques, amphotères, zwittérioniques ou leurs mélanges ; des agents épaississants minéraux ; des agents antioxydants ; des agents de pénétration ; des agents séquestrants ; des parfums ; des tampons ; des agents dispersants ; des agents de conditionnement tels que par exemple des cations, des polymères cationiques ou amphotères, les chitosanes, les silicones volatiles ou non volatiles, modifiées ou non modifiées ; des agents filmogènes ; des céramides ; des agents conservateurs ; des agents stabilisants ; des agents opacifiants.

Les adjuvants ci-dessus sont en général présents en quantité comprise pour chacun d'eux entre 0,01 et 20 % en poids par rapport au poids de la composition.

De préférence, la composition selon l'invention comprend au moins un polymère.

La composition selon l'invention peut se présenter sous des formes diverses, telles que sous forme de liquides, de shampooings, de crèmes, de gels, ou sous toute autre forme appropriée.

Un autre objet de l'invention est constitué par un procédé de traitement de fibres kératiniques humaines dans lequel on applique la composition selon l'invention, sur lesdites fibres.

Selon une première variante, on applique sur lesdites fibres, sèches ou humides, une composition telle que définie, pendant un temps suffisant, après quoi on rince, habituellement à l'eau, on lave éventuellement au shampooing, on rince à nouveau et on sèche ou on laisse sécher les fibres résultantes.

Selon une deuxième variante du procédé, on applique sur lesdites fibres, sèches ou humides, une composition telle que définie sans rinçage final.

La première variante est utilisable pour tout type de compositions, que celles-ci comprennent ou non, un agent oxydant et/ou un colorant direct additionnel et/ou une base d'oxydation éventuellement associée à un coupleur.

La seconde variante est particulièrement appropriée pour des compositions ne comprenant pas de colorant d'oxydation (base d'oxydation et éventuellement coupleur) ni d'agent oxydant.

Dans le cas de la première variante du procédé, le temps d'application est habituellement suffisant pour développer la coloration et/ou l'éclaircissement souhaité(s).

A titre indicatif, la durée d'application de la composition est d'environ 1 à 60 minutes et plus particulièrement d'environ 2 à 30 minutes.

Par ailleurs, la température à laquelle le procédé selon l'invention est mis en oeuvre, est généralement comprise entre la température ambiante (15 à 25°C) et 60°C et plus particulièrement entre 15 et 45 °C.

Au cas où la composition comprend un agent oxydant, le procédé selon l'invention comporte une étape préliminaire consistant à stocker sous forme séparée, d'une part, une composition comprenant, dans un milieu cosmétiquement acceptable, au moins un colorant direct cationique comprenant au moins une liaison triple carbone-carbone et plus particulièrement au moins un colorant de formules (I), (II) ou (III), éventuellement au moins un colorant direct additionnel et/ou éventuellement au moins une base d'oxydation éventuellement associée à au moins un coupleur, et d'autre part, une composition comprenant, dans un milieu cosmétiquement acceptable, au moins un agent oxydant, puis à procéder à leur mélange au moment de l'emploi. Il est à noter que le(s) colorant(s) direct(s) selon l'invention, le(s) colorant(s) direct(s) additionnel(s) et la ou les bases et éventuellement le ou les coupleurs peuvent être stockés séparément les uns des autres ou partiellement combinés (les colorants directs ensembles ou bien le(s) colorant(s) direct(s) additionnel(s) avec la ou les bases et/ou le ou les coupleurs par exemple).

Une fois le mélange réalisé, le procédé selon l'invention est mis en oeuvre conformément à ce qui a été mentionné auparavant.

Un autre objet de l'invention est un dispositif à plusieurs compartiments, comprenant au moins un compartiment renfermant une composition comprenant au moins un colorant direct cationique comprenant au moins une liaison triple carbone-carbone et plus particulièrement au moins un colorant de formules (I), (II) ou (III), et au moins un autre compartiment renfermant une composition comprenant au moins un agent oxydant.

Ce dispositif peut être équipé d'un moyen permettant de délivrer sur les cheveux le mélange souhaité, tel que les dispositifs décrits dans le brevet FR 2586913.

A noter qu'au cas où la composition renferme au moins un colorant direct additionnel et/ou au moins une base d'oxydation éventuellement associée à au moins un coupleur (précurseur(s) de colorant d'oxydation), ce ou ces composés peuvent se trouver, dans le même compartiment que celui du ou des colorants cationiques comprenant au moins une liaison triple carbone-carbone et plus avantageusement au moins un colorant de formules (I), (II) et/ou (III), ou bien dans un troisième compartiment, ou encore, chacun dans un compartiment différent. On pourrait aussi envisager de mettre les colorants directs selon l'invention avec le(s) colorant(s) direct(s) additionnel(s) dans un même compartiment et le(s) précurseur(s) de colorant(s) d'oxydation (base(s) et éventuellement coupleur(s)) dans un compartiment différent.

Les exemples qui suivent sont destinés à illustrer l'invention sans pour autant en limiter la portée.

### EXEMPLE 1

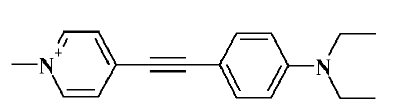

Le composé, dont la structure est donnée ci-dessus, est mis en oeuvre dans la formulation suivante :

| | |
|---|---|
| Colorant | 0,5% |
| Alkyl (C8/C10 50/50) polyglucoside | 5% |
| PEG-8 | 6% |
| Alcool benzylique | 4% |
| Hydroxyéthyl cellulose (PM 720.000) | 2% |
| Tampon pH9 | 50% |
| Eau | QSP 100 |

Ce mélange est appliqué 30 minutes à température ambiante sur des mèches de cheveux 90% blancs naturels et 90% blancs permanentés.

Après l'application, les mèches sont rincées, shampouinées puis séchées. Elles sont colorées en cuivré intense.

Même en réduisant le temps de pause à 5 minutes, on obtient encore une nuance suffisamment puissante.

Des formulations analogues ont été réalisées en remplaçant le tampon pH9 par des tampons pH4 et pH7.

On obtient à chaque fois une couleur cuivré intense.

Ils présentent une bonne montée également en milieu éclaircissant avec une bonne ténacité lumière.

### EXEMPLE 2

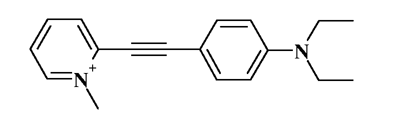

Le composé, dont la structure est donnée ci-dessus, est mis en oeuvre dans la formulation suivante :

| | |
|---|---|
| Colorant | 0.5% |
| Alkyl (C8/C10 50/50) polyglucoside | 5% |
| PEG-8 | 6% |
| Alcool benzylique | 4% |
| Hydroxyéthyl cellulose (PM 720.000) | 2% |
| Tampon pH9 | 50% |
| Eau | QSP 100 |

Ce mélange est appliqué 30 minutes à température ambiante sur des mèches de cheveux 90% blancs naturels et 90% blancs permanentés.

Après l'application, les mèches sont rincées, shampouinées puis séchées.

Elles sont colorées en cuivré doré intense.

Même en réduisant le temps de pause à 5 minutes, on obtient encore une nuance suffisamment puissante.

Des formulations analogues ont été réalisées en remplaçant le tampon pH9 par des tampons pH4 et pH7.

On obtient à chaque fois une couleur cuivré intense.

Ils présentent une bonne montée également en milieu éclaircissant avec une bonne ténacité lumière.

## Revendications

1. Composition de coloration de fibres kératiniques humaines comprenant, dans un milieu cosmétiquement acceptable, un ou plusieurs colorants directs cationiques comprenant au moins une liaison triple carbone-carbone

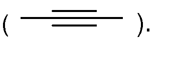

2. Composition selon la revendication précédente, **caractérisée en ce qu'**elle comprend, dans un milieu cosmétiquement acceptable, un ou plusieurs colorants directs de formules (I), (II) ou (III) suivantes : 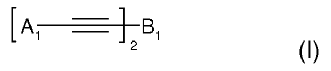 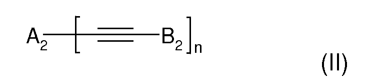 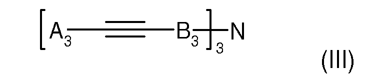 Formules dans lesquelles :
- A₁, A₂, A₃, indépendamment les uns des autres, correspondent à un groupement hétérocyclique cationique rattaché à la fonction C≡C ou aux fonctions C≡C au moyen de l'un des atomes de carbone de l'hétérocycle ou du noyau aromatique avec lequel l'hétérocycle est éventuellement condensé ;
- A₁, A₂, A₃, étant éventuellement substitués, en dehors des radicaux portés par les atomes d'azote quaternisés, par un ou plusieurs radicaux, identiques ou non, choisis parmi les atomes d'halogène comme le fluor ou le chlore, les radicaux alkyle en C₁-C₂₅, les radicaux alcoxy (C₁-C₄), les radicaux alkyle(C₁-C₂₀)carbonylamino, les radicaux phényle ;
- le ou les atomes d'azote quaternisés de l'hétérocycle, lorsqu'il sont présents, sont éventuellement porteur(s) d'un radical amino ; d'un radical acyle en C₂-C₂₀ ; d'un radical alkyle en C₁-C₂₅ éventuellement substitué par au moins un radical phényle, phényl-carbonyle, alcoxy(C₁-C₄)carbonyle, sulfo (-SO₃M avec M représentant un atome d'hydrogène, un métal alcalin), éthylényle (-CH=CH₂), acétylényle (-C≡CH) ou phényle-acétylényle (-C≡C-C₆H₅) ;
- B₁, B₂, B₃, indépendamment les uns des autres, correspondent à un noyau aromatique choisi parmi les groupements phényle, anthracényle, naphtényle, rattaché à une ou deux fonctions C≡C ;
- B₁, B₂, B₃, étant éventuellement substitués par un ou plusieurs groupements amino éventuellement porteurs d'un ou deux radicaux identiques ou non choisis parmi les radicaux alkyles en C₁-C₁₈, alkyl(C₁-C₂₀)carbonyle ; alkyl(C₁-C₂₀)sulfonyle, phényle éventuellement substitué par un groupement alcoxy en C₁-C₄ ; hydroxyle ; alcoxy en C₁-C₄; alkyle en C₁-C₁₀ éventuellement porteur d'un groupement amino ; phénoxy; sulfo (-SO₃M avec M représentant un atome d'hydrogène, un métal alcalin) ; cyano ; trifluorométhyle ; halogène choisi parmi le chlore, le fluor et le brome ; acétylthio (CH₃-CO-S-) ;
- n est égal à 1 ou 2 ;
- l'électroneutralité des composés étant assurée, si nécessaire, au moyen d'un ou plusieurs anions, identiques ou différents, cosmétiquement acceptables.

3. Composition selon la revendication précédente, **caractérisée en ce que** les groupements A₁, A₂, A₃, indépendamment les uns des autres, correspondent à un groupement hétérocyclique cationique choisi parmi les groupements pyridinium, (benz)imidazolium, (benzo)thiazolium, benzoxazolium, (iso)quinolinium, (benz)indolium, acridinium, thioxanthilium, quinolizinium, phénazinium, pyrrolopyridinium, rattaché à la fonction C≡C ou aux fonctions C≡C au moyen de l'un des atomes de carbone de l'hétérocycle ou du noyau aromatique avec lequel l'hétérocycle est éventuellement condensé.

4. Composition selon l'une quelconque des revendications précédentes, **caractérisée en ce que** le colorant direct est de formule (I), et le groupement A₁ est un groupement phénazinium.

5. Composition selon la revendication précédente, **caractérisée en ce que** le groupement B₁ est un groupement anthracényle.

6. Composition selon l'une quelconque des revendications 1 ou 2, **caractérisée en ce que** le colorant direct est de formule (II), n vaut 1 et le groupement A₂ est choisi parmi les groupements pyridinium, benzimidazolium, (benzo)thiazolium, benzoxazolium, (iso)quinolinium, (benz)indolium, acridinium, thioxanthilium, quinolizinium, pyrrolopiridinium, éventuellement substitués.

7. Composition selon la revendication précédente, **caractérisée en ce que** le colorant direct est de formule (II), n vaut 2 et le groupement A₂ est un groupement pyridinium.

8. Composition selon l'une des revendications précédentes, **caractérisée en ce que** les groupements B₂, indépendamment les uns des autres, correspondent à un groupement phényle, éventuellement substitué, naphtalényle.

9. Composition selon la revendication précédente, **caractérisée en ce que** le colorant direct est de formule (III) et les groupements A₃ sont des groupements pyridinium.

10. Composition selon la revendication précédente, **caractérisée en ce que** les groupements B₃ sont des groupements phényle.

11. Composition selon l'une des revendications précédentes, **caractérisée en ce que** le colorant direct de formules (I), (II) ou (III) sont choisis parmi les composés suivants, seuls ou en mélanges :
| | |
|---|---|
| 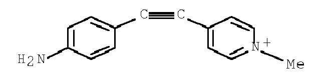 | 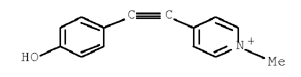 |
| Sel de pyridinium, 4-[(4-aminophenyl)ethynyl]-1-methyl | Sel de pyridinium, 4-[(4-hydroxyphenyl)ethynyl]-1-methyl |
| 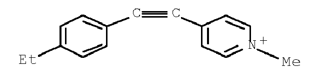 | 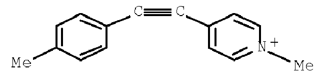 |
| Sel de pyridinium, 4-[(4-ethylphenyl)ethynyl]-1-methyl- | Sel de pyridinium, 1-methyl-4-[(4-methylphenyl)ethynyl] |
| 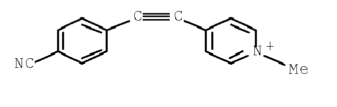 | 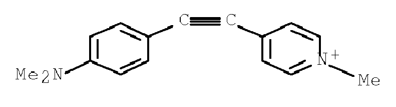 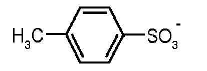 |
| Sel de pyridinium, 4-[(4-cyanophenyl)ethynyl]-1-methyl | Sel d'acide 4-méthylbenzènesulfonique (1:1) et de pyridinium, 4-[[4-(dimethylamino)phenyl]ethynyl]-1-methyl-, monohydraté |
| | 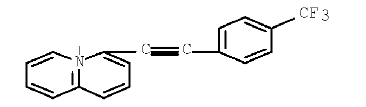 |
| | Sel de quinolizinium, 4-[[4-(trifluoromethyl)phenyl]ethynyl]- |
| 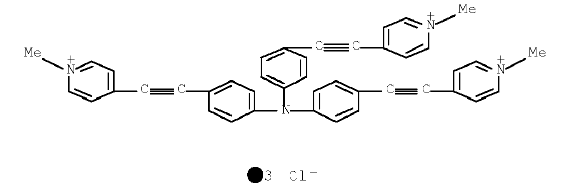 | |
| Trichlorure de pyridinium, 4,4',4"-[nitrilotris(4,1-phenylene-2,1-ethynediyl)]tris[1-methyl-, trichloride | |
| 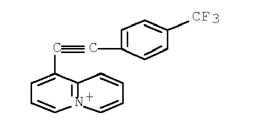 | 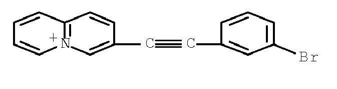 |
| Sel de quinolizinium, 1-[[4-(trifluoromethyl)phenyl]ethynyl]- | Sel de quinolizinium, 3-[(3-bromophenyl)ethynyl]- |
| 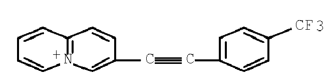 | 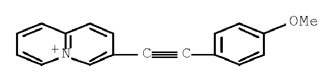 |
| Sel de quinolizinium, 3-[[4-(trifluoromethyl)phenyl]ethynyl]- | Sel de quinolizinium, 3-[(4-methoxyphenyl)ethynyl]- |
| 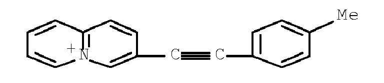 | 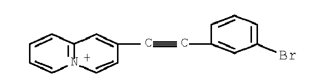 |
| Sel de quinolizinium, 3-[(4-methylphenyl)ethynyl]- | Sel de quinolizinium, 2-[(3-bromophenyl)ethynyl]- |
| 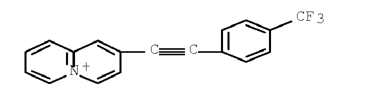 | 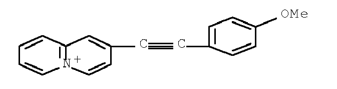 |
| Sel de quinolizinium, 2-[[4-(trifluoromethyl)phenyl]ethynyl] | Sel de quinolizinium, 2-[(4-methoxyphenyl)ethynyl] |
| 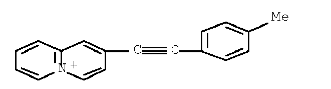 | 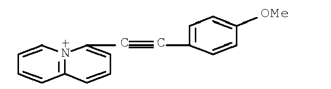 |
| Sel de quinolizinium, 2-[(4-methylphenyl)ethynyl]- | Sel de quinolizinium, 4-[(4-methoxyphenyl)ethynyl]- |
| 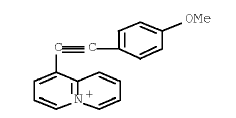 | 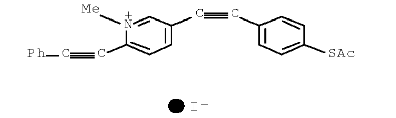 |
| Sel de quinolizinium, 1-[(4-methoxyphenyl)ethynyl]- | Iodure de pyridinium, 5-[[4-(acetylthio)phenyl]ethynyl]-1-methyl-2-(phenylethynyl) |
| 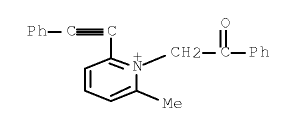 | 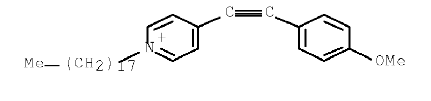 |
| Sel de pyridinium, 2-methyl-1-(2-oxo-2-phenylethyl)-6-(phenylethynyl)- | Sel de pyridinium, 4-[(4-methoxyphenyl)ethynyl]-1-octadecyl- |
| 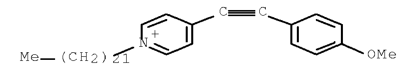 | 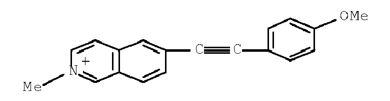 |
| Sel de pyridinium, 1-docosyl-4-[(4-methoxyphenyl)ethynyl]- | Sel d'isoquinolinium, 6-[(4-methoxyphenyl)ethynyl]-2-methyl |
| 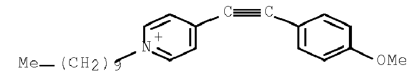 | 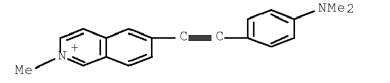 |
| Sel de pyridinium, 1-decyl-4-[(4-methoxyphenyl)ethynyl]- | Sel d'isoquinolinium, 6-[[4-(dimethylamino)phenyl]ethynyl]-2-methyl |
| 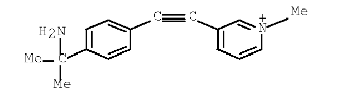 | 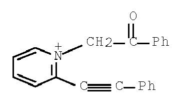 |
| Sel de pyridinium, 3-[[4-(1-amino-1-methylethyl)phenyl]ethynyl]-1-methyl | Sel de pyridinium, 1-(2-oxo-2-phenylethyl)-2-(phenylethynyl)- |
| 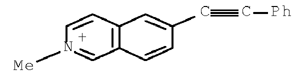 | 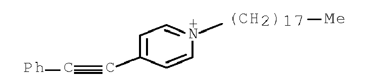 |
| Sel d'isoquinolinium, 2-methyl-6-(phenylethynyl)- | Sel de pyridinium, 1-octadecyl-4-(phenylethynyl)- |
| 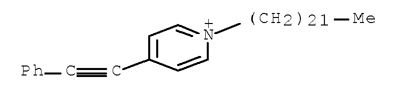 | 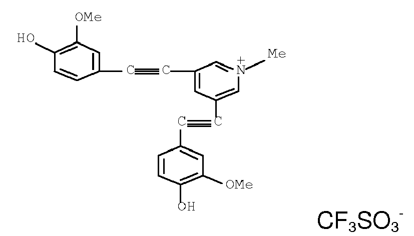 |
| Sel de pyridinium, 1-docosyl-4-(phenylethynyl)- | Sel d'acide trifluorométhanesulfonique (1:1) et de pyridinium, 3,5-bis[(4-hydroxy-3-methoxyphenyl)ethynyl]-1-methyl |
| 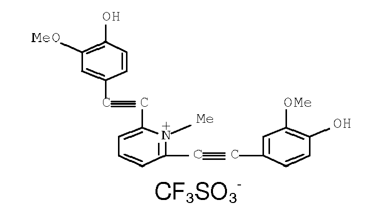 | 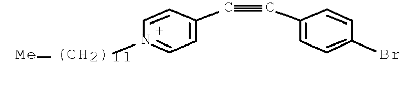 |
| Sel d'acide trifluorométhanesulfonique (1:1) et de pyridinium, 2,6-bis[(4-hydroxy-3-methoxyphenyl)ethynyl]-1-methyl | Sel de pyridinium, 4-[(4-bromophenyl)ethynyl]-1-dodecyl |
| 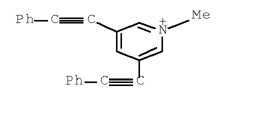 | 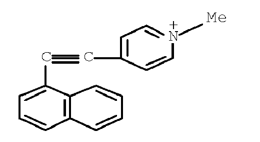 |
| Sel de pyridinium, 1-methyl-3,5-bis(phenylethynyl)- | Sel de pyridinium, 1-methyl-4-(1-naphthalenylethynyl) |
| 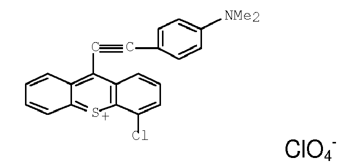 | 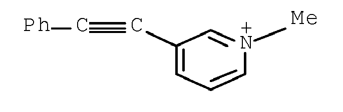 |
| Perchlorate de thioxanthylium, 4-chloro-9-[[4-(dimethylamino)phenyl]ethynyl] | Sel de pyridinium, 1-methyl-3-(phenylethynyl)- |
| 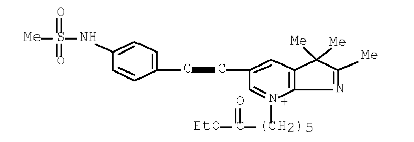 | 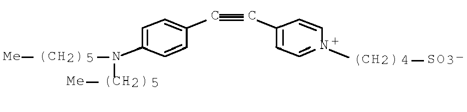 |
| Sel de 3H-Pyrrolo[2,3-b]pyridinium, 7-(6-ethoxy-6-oxohexyl)-2,3,3-trimethyl-5-[[4-[(methylsulfonyl)amino]phenyl]ethynyl | Sel interne de pyridinium, 4-[[4-(dihexylamino)phenyl]ethynyl]-1-(4-sulfobutyl) |
| 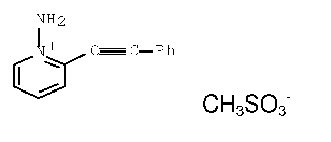 | 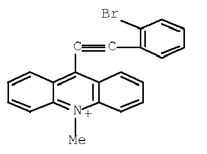 |
| Méthanesulfonate de pyridinium, 1-amino-2-(phenylethynyl) | Sel d'acridinium, 9-[(2-bromophenyl)ethynyl]-10-methyl |
| 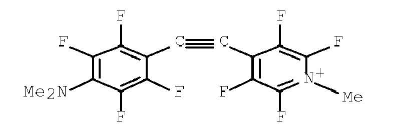 | 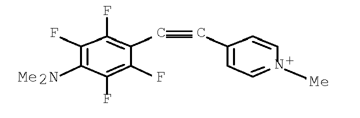 |
| Sel de pyridinium, 4-[[4-(dimethylamino)-2,3,5,6-tetrafluorophenyl]ethynyl]- 2,3,5,6-tetrafluoro-1-methyl | Sel de pyridinium, 4-[[4-(dimethylamino)-2,3,5,6-tetrafluorophenyl]ethynyl]-1-methyl- |
| 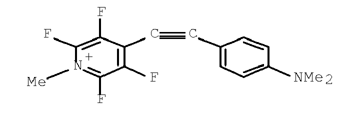 | 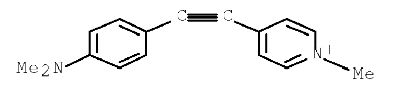 |
| Sel de pyridinium, 4-[[4-(dimethylamino)phenyl]ethynyl]-2,3,5,6-tetrafluoro-1-methyl- | Sel de pyridinium, 4-[[4-(dimethylamino)phenyl]ethynyl]-1-methyl- |
| 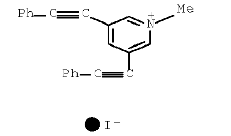 | 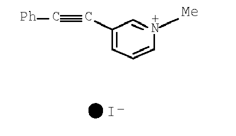 |
| Iodure de pyridinium, 1-methyl-3,5-bis(phenylethynyl) | Iodure de pyridinium, 1-methyl-3-(phenylethynyl) |
| 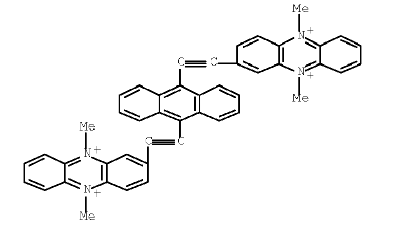 | 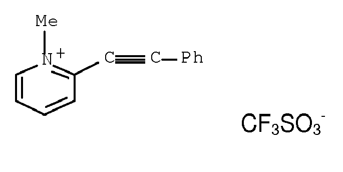 |
| Sel de phenazinium, 2,2'-(9,10-anthracenediyldi-2,1-ethynediyl)bis[5,10-dimethyl | Sel d'acide trifluorométhanesulfonique et de pyridinium, 1-methyl-2-(phenylethynyl) |
| 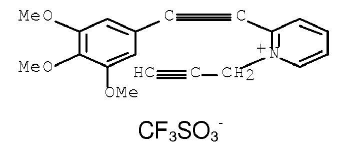 | 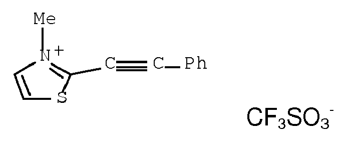 |
| Sel d'acide trifluoromethanesulfonique (1 :1) et de pyridinium, 1-(2-propynyl)-2-[(3,4,5-trimethoxyphenyl)ethynyl] | Sel d'acide trifluoromethanesulfonique (1:1) et de thiazolium, 3-methyl-2-(phenylethynyl) |
| 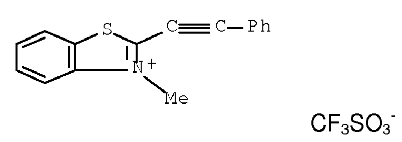 | 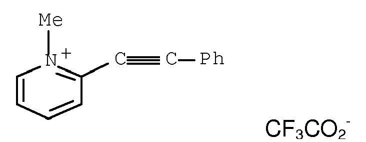 |
| Sel d'acide trifluorométhanesulfonique (1:1) et de benzothiazolium, 3-methyl-2-(phenylethynyl) | Sel d'acide trifluoroacétique (1:1) et de pyridinium, 1-methyl-2-(phenylethynyl) |
| 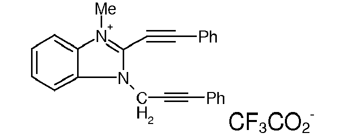 | 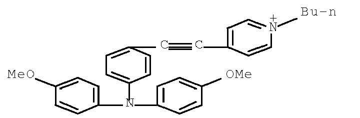 |
| sel d'acide trifluoroacétique (1:1) et de 1H-Benzimidazolium, 1-methyl-2-(phenylethynyl)-3-(3-phenyl-2-propynyl) | Sel de pyridinium, 4-[[4-[bis(4-methoxyphenyl)amino]phenyl]ethynyl]-1-butyl- |
| 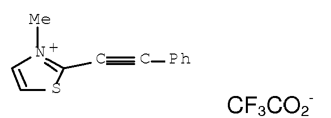 | 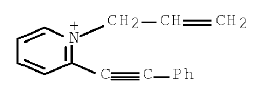 |
| Sel d'acide trifluoroacétique (:1) et de thiazolium, 3-methyl-2-(phenylethynyl) | Sel de pyridinium, 2-(phenylethynyl)-1-(2-propenyl)- |
| 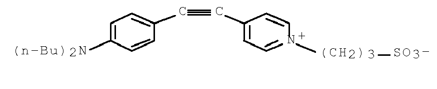 | 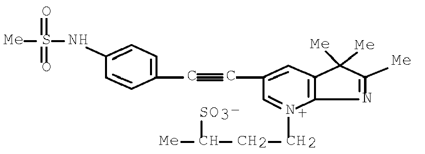 |
| Sel interne de pyridinium, 4-[[4-(dibutylamino)phenyl]ethynyl]-1-(3-sulfopropyl) | Sel interne de 3H-Pyrrolo[2,3-b]pyridinium, 2,3,3-trimethyl-5-[[4-[(methylsulfonyl)amino]phenyl]ethynyl]-7-(3-sulfobutyl) |
| 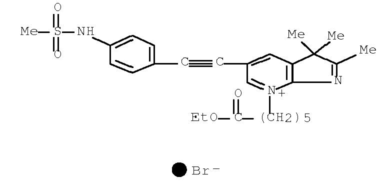 | 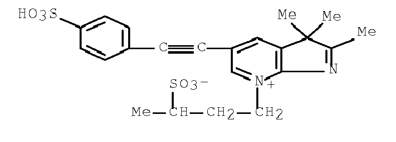 |
| Bromure de 3H-Pyrrolo[2,3-b]pyridinium, 7-(6-ethoxy-6-oxohexyl)-2,3,3-trimethyl-5-[[4-[(methylsulfonyl)amino]phenyl]ethynyl]-, | Sel interne de 3H-Pyrrolo[2,3-b]pyridinium, 2,3,3-trimethyl-7-(3-sulfobutyl)-5-[(4-sulfophenyl)ethynyl] |
| 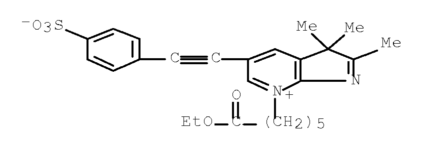 | 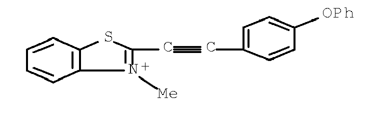 |
| Sel interne de 3H-Pyrrolo[2,3-b]pyridinium, 7-(6-ethoxy-6-oxohexyl)-2,3,3-trimethyl-5-[(4-sulfophenyl)ethynyl] | Sel de benzothiazolium, 3-methyl-2-[(4-phenoxyphenyl)ethynyl]- |
| 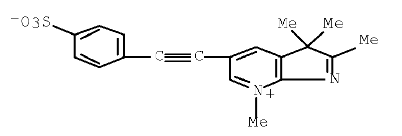 | 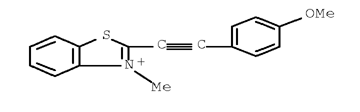 |
| Sel interne de 3H-Pyrrolo[2,3-b]pyridinium, 2,3,3,7-tetramethyl-5-[(4-sulfophenyl)ethynyl] | Sel de benzothiazolium, 2-[(4-methoxyphenyl)ethynyl]-3-methyl |
| 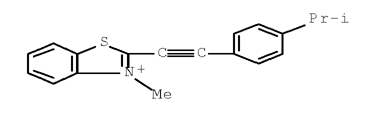 | 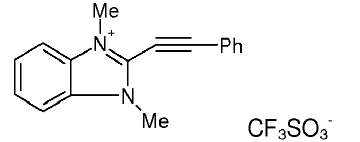 |
| Sel de benzothiazolium, 3-methyl-2-[[4-(1-methylethyl)phenyl]ethynyl]- | Sel d'acide trifluorométhanesulfonique acid (1:1) et de 1H-Benzimidazolium, 1,3-dimethyl-2-(phenylethynyl) |
| 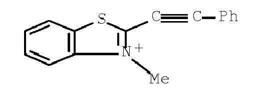 | 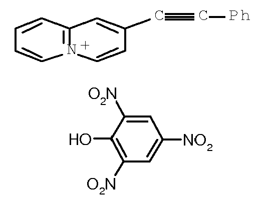 |
| Sel de benzothiazolium, 3-methyl-2-(phenylethynyl)- | Sel de 2,4,6-trinitrophenol (1:1) et de quinolizinium, 2-(phenylethynyl) |
| 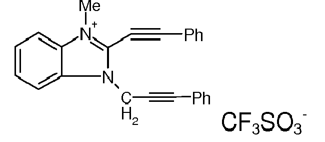 | 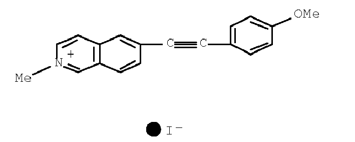 |
| Sel d'acide trifluoromethanesulfonique (1:1) 1H-Benzimidazolium, 1-methyl-2-(phenylethynyl)-3-(3-phenyl-2-propynyl) | Iodure d'Isoquinolinium, 6-[(4-methoxyphenyl)ethynyl]-2-methyl |
| 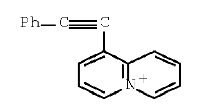 | 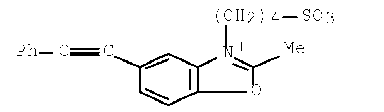 |
| Sel de 2,4,6-trinitrophenol (1:1) et de quinolizinium, 1-(phenylethynyl) | Sel interne de benzoxazolium, 2-methyl-5-(phenylethynyl)-3-(4-sulfobutyl) |
| 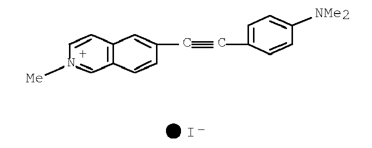 | 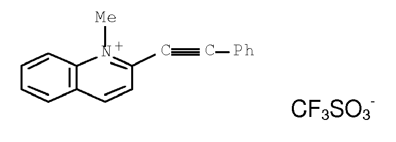 |
| Iodure d'isoquinolinium, 6-[[4-(dimethylamino)phenyl]ethynyl]-2-methyl | Sel d'acide trifluorométhanesulfonique (1:1) et de quinolinium, 1-methyl-2-(phenylethynyl) |
| 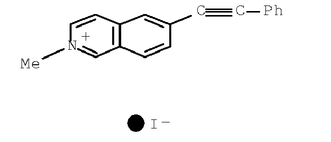 | 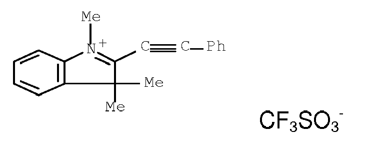 |
| Iodure d'isoquinolinium, 2-methyl-6-(phenylethynyl) | Sel d'acide trifluoromethanesulfonique (1:1) et de 3H-Indolium, 1,3,3-trimethyl-2-(phenylethynyl) |
| 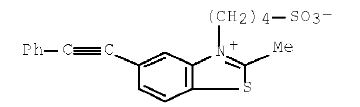 | 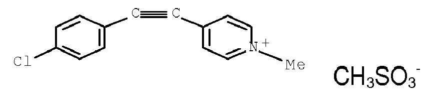 |
| Sel interne de benzothiazolium, 2-methyl-5-(phenylethynyl)-3-(4-sulfobutyl) | Méthylsulfate de pyridinium, 4-[(4-chlorophenyl)ethynyl]-1-methyl-, methyl sulfate |
| 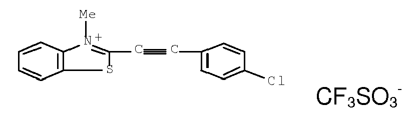 | 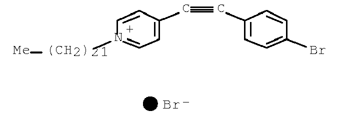 |
| Sel d'acide trifluorométhanesulfonique (1:1) et de b Benzothiazolium, 2-[(4-chlorophenyl)ethynyl]-3-methyl-, | Bromure de pyridinium, 4-[(4-bromophenyl)ethynyl]-1-docosyl |
| 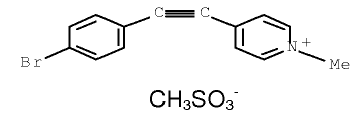 | 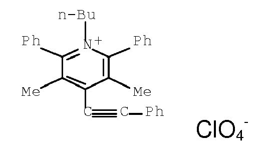 |
| Méthylsulfate de pyridinium, 4-[(4-bromophenyl)ethynyl]-1-methyl- | Perchlorate de pyridinium, 1-butyl-3,5-dimethyl-2,6-diphenyl-4-(phenylethynyl) |
| 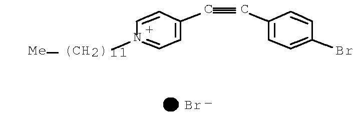 | 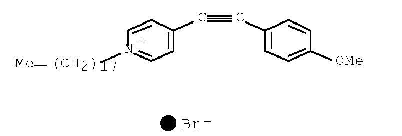 |
| Bromure de pyridinium, 4-[(4-bromophenyl)ethynyl]-1-dodecyl | Bromure de pyridinium, 4-[(4-methoxyphenyl)ethynyl]-1-octadecyl |
| 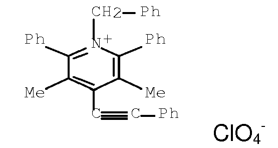 | 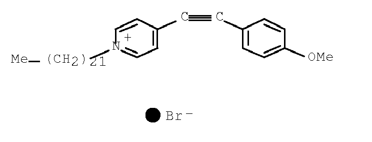 |
| Perchlorate de pyridinium, 3,5-dimethyl-2,6-diphenyl-4-(phenylethynyl)-1-(phenylmethyl) | Bromure de pyridinium, 1-docosyl-4-[(4-methoxyphenyl)ethynyl] |
| 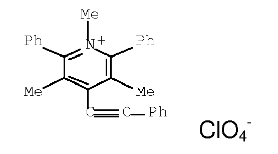 | 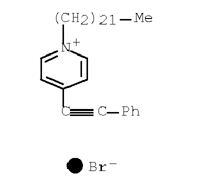 |
| Perchlorate de pyridinium, 1,3,5-trimethyl-2,6-diphenyl-4-(phenylethynyl) | Bromure de pyridinium, 1-docosyl-4-(phenylethynyl) |
| 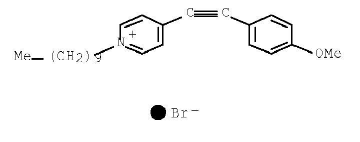 | 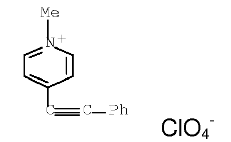 |
| Bromure de pyridinium, 1-decyl-4-[(4-methoxyphenyl)ethynyl] | Perchlorate de 1-Methyl-4-phenylethynylpyridinium |
| 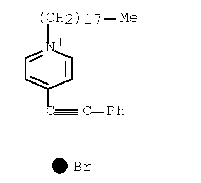 | 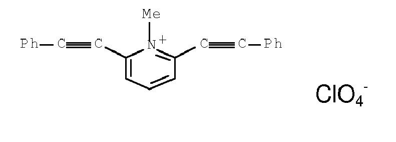 |
| Bromure de pyridinium, 1-octadecyl-4-(phenylethynyl) | Perchlorate de 1-Methyl-2,6-bis(phenylethynyl)pyridinium |
| 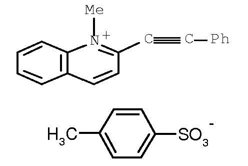 | 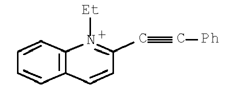 |
| Paratoluènesulfonate de 1-Methyl-2-phenylethynylquinolinium | Perchlorate de 1-Ethyl-2-phenylethynylquinolinium |
| 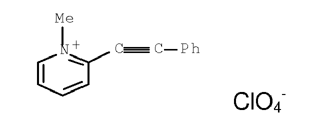 | 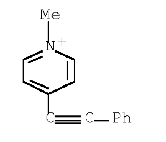 |
| Perchlorate de 1-Methyl-2-phenylethynylpyridinium | Sel de 1-Methyl-4-phenylethynylpyridinium methyl |
| 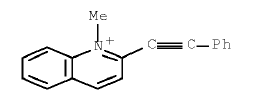 | 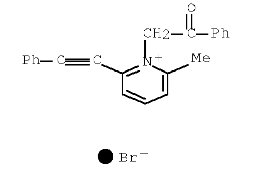 |
| Perchlorate de 1-Methyl-2-phenylethynylquinolinium | Bromure de pyridinium, 2-methyl-1-(2-oxo-2-phenylethyl)-6-(phenylethynyl) |
| 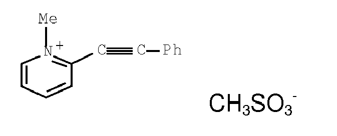 | 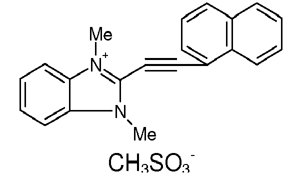 |
| Méthylsulfate de -Methyl-2-phenylethynylpyridinium | Méthylsulfate de 1H-Benzimidazolium, 1,3-dimethyl-2-(1-naphthalenylethynyl) |
| 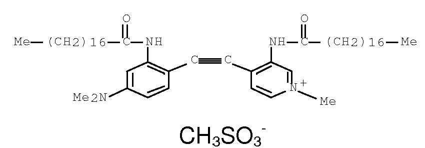 | 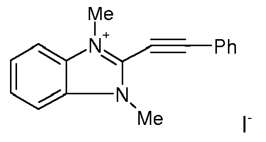 |
| Methyl sulfate de pyridinium, 4-[[4-(dimethylamino)-2-[(1-oxooctadecyl)amino]phenyl]ethynyl]-1-methyl-3-[(1-oxooctadecyl)amino] | Iodure de benzimidazolium, 1,3-dimethyl-2-(phenylethynyl)-, |
| 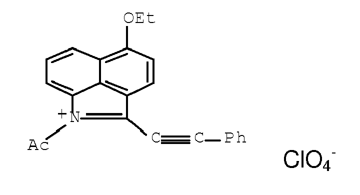 | 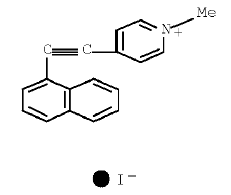 |
| Perchlorate de benz[cd]indolium, 1-acetyl-5-ethoxy-2-(phenylethynyl) | lodure de pyridinium, 1-methyl-4-(1-naphthalenylethynyl)-, |
| 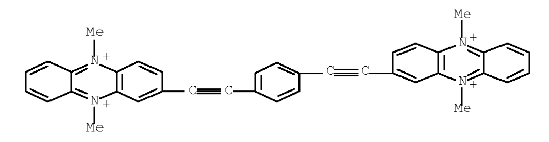 | |
| Sel de phenazinium, 2,2'-(1,4-phenylenedi-2,1-ethynediyl)bis[5,10-dimethyl- | |

12. Composition selon l'une quelconque des revendications précédentes, **caractérisée en ce que** la teneur en colorant(s) direct(s) de formules (I), (II) ou (III) est comprise entre 0,01 et 20% en poids par rapport au poids total de la composition.

13. Composition selon l'une quelconque des revendications précédentes, **caractérisée en ce qu'**elle renferme au moins un agent oxydant.

14. Procédé de traitement de fibres kératiniques plus particulièrement de fibres kératiniques humaines, **caractérisé en ce que** l'on applique sur lesdites fibres, sèches ou humides, une composition telle que définie selon l'une quelconque des revendications précédentes.

15. Dispositif à plusieurs compartiments pour la teinture et l'éclaircissement des cheveux, comprenant au moins un compartiment renfermant une composition selon l'une quelconque des revendications 1 à 12, et au moins un autre compartiment renfermant une composition comprenant au moins un agent oxydant.
